# FASCICULE DE BREVET EUROPEEN

(11) **EP 2 143 417 B1**
(45) Date de publication et mention de la délivrance du brevet: **26.11.2014**
(21) Numéro de dépôt: 09163371.9
(22) Date de dépôt: 22.06.2009
(51) Int. Cl.: A61K 8/44, A61K 8/46, A61K 8/898, A61Q 5/02, A61Q 5/00

(54) **Compositions cosmétiques détergentes comprenant une silicone aminée et utilisation**
Reinigende kosmetische Zusammensetzungen enthaltend ein aminosilikon und deren Verwendung
Detergent cosmetic compositions comprising an amino silicone, and use thereof

(30) Priorité: 08.07.2008 FR 0854633
(43) Date de publication de la demande: 13.01.2010
(73) Titulaire: L'Oréal, 75008 Paris (FR)
(72) Inventeur: Decoster, Sandrine, 95210, Saint Gratien (FR); Naplaz, Stéphanie, 75011, Paris (FR)
(74) Mandataire: Le Blainvaux Bellegarde, Françoise

(56) Documents cités:
- EP-A1- 1 543 820
- EP-A1- 1 726 294
- EP-A1- 1 726 295
- EP-A1- 1 849 453
- EP-A2- 1 321 131
- US-A1- 2007 041 930
- ANONYMOUS: "Cosmetic Raw Materials" CLARIANT PERSONAL CARE BROCHURE, [Online] 2006, - 2001 page 39, XP002517678 Extrait de l'Internet: URL:http://fun.clariant.com/C125691A003596 E5/vwLookupDownloads/product_range_060922. pdf/$FILE/product_range_060922.pdf> [extrait le 2009-03-04]
- 'Rhodorsil 21645 Safety Datasheet', [en ligne] 10 Août 2001, XP055061993 Extrait de l'Internet: <URL:http://www.nicnas.gov.au/publications/ car/new/na/nafullr/na0800fr/na819fr.pdf> [extrait le 2013-05-06]

## Description

La présente invention concerne de nouvelles compositions cosmétiques à propriétés améliorées destinées au nettoyage et à la protection de la couleur des fibres kératiniques telles que les cheveux, et comprenant, dans un support aqueux cosmétiquement acceptable, au moins un tensioactif anionique sulfate ou sulfonate, au moins un tensioactif amphotère et au moins une silicone aminée particulière, avec un rapport particulier en poids tensioactif amphotère / tensioactif anionique sulfate ou sulfonate. L'invention concerne aussi l'utilisation desdites compositions pour protéger la couleur artificielle des cheveux teints.

Il est connu de teindre les fibres kératiniques notamment humaines et en particulier les cheveux avec des compositions tinctoriales comprenant des précurseurs de colorant d'oxydation, appelés généralement bases d'oxydation. Ces bases d'oxydation sont des composés incolores ou faiblement colorés qui, associés à des produits oxydants, donnent naissance par un processus de condensation oxydative à des composés colorés. On sait également que l'on peut faire varier les nuances obtenues avec ces bases d'oxydation en les associant à des coupleurs ou modificateurs de coloration. La variété des molécules mises en jeu au niveau des bases d'oxydation et des coupleurs permet l'obtention d'une riche palette de couleurs.

Il est aussi connu de teindre les fibres kératiniques par une coloration directe. Le procédé classiquement utilisé en coloration directe consiste à appliquer sur les fibres kératiniques des colorants directs qui sont des molécules colorées et colorantes ayant une affinité pour les fibres, à laisser poser, puis à rincer les fibres.

Les colorations qui en résultent sont des colorations particulièrement chromatiques qui sont cependant temporaires ou semi-permanentes car la nature des interactions qui lient les colorants directs à la fibre kératinique et leur désorption de la surface et/ou du coeur de la fibre sont responsables de leur faible puissance tinctoriale et de leur mauvaise tenue aux lavages.

La couleur artificielle des cheveux apportée par un traitement de coloration directe ou d'oxydation s'estompe progressivement du fait des lavages répétés et conduit dans le temps à un affadissement de la coloration des cheveux. L'utilisation des produits soins rincés et non rincés commercialisés n'améliore pas suffisamment la tenue de la couleur artificielle des cheveux.

Il est donc nécessaire de mettre au point des moyens permettant de protéger la couleur artificielle de l'effet des lavages répétés, ceci dans des conditions douces compatibles avec les cheveux colorés, notamment en terme de température.

Il existe donc un besoin d'améliorer la ténacité des colorations directes ou d'oxydation, en particulier vis à vis des shampooings.

On a déjà utilisé dans ce but des silicones aminées telles que décrite dans le document EP1312346. Cependant, les compositions divulguées ne donnent pas encore satisfaction.

Il est décrit dans le document EP1726295 des compositions de lavage des matières kératiniques, comprenant des tensioactifs anioniques de type sulfate et/ou sulfonate, des tensioactifs anioniques carboxyliques et des tensioactifs amphotères, en mélange avec des silicones aminées comprenant une fonction amine portée par un groupe stériquement encombré.

Le document US2007/0041930 est relatif à l'utilisation dans des compositions capillaires, de polysiloxanes particuliers portant des groupements ammonium quaternaire, en particulier pour apporter du conditionnement aux cheveux, pour améliorer la stabilité de la couleur des cheveux teints, et pour protéger les cheveux de la chaleur et des UV.

Après d'importantes études sur la question, la demanderesse a découvert de manière tout à fait inattendue et surprenante que l'utilisation, d'une composition détergente comprenant une association particulière de tensioactif et d'au moins une silicone aminée particulière, permettait de protéger la couleur artificielle de fibres kératiniques vis-à-vis des lavages répétés et d'améliorer la ténacité de la coloration.

Cette découverte est à l'origine de la présente invention.

On a en effet constaté qu'en utilisant la composition selon l'invention, on limitait l'affadissement de la coloration après plusieurs shampooings. Ainsi, la perte de coloration, évaluée par exemple à partir de la variation de la valeur du coefficient DL* des mèches avant et après shampooings dans le système CIE L*a*b* ou également à partir de DE* (correspondant à la racine carrée de la somme des carrés des variations des coefficients L*, a* et b* des mèches avant et après shampooings) était diminuée.

Les compositions conformes à l'invention confèrent également aux matières kératiniques notamment les cheveux, un remarquable effet traitant qui se manifeste notamment par une facilité de démêlage, ainsi qu'un apport de volume, de légèreté, de lissage, de douceur et de souplesse sans aucun effet de charge.
Les cheveux ont un aspect naturel, propre et non gras.

Ainsi, la présente invention a pour objet de nouvelles compositions cosmétiques détergentes caractérisée par le fait qu'elles comprennent, dans un milieu aqueux cosmétiquement acceptable,
(A) un ou plusieurs tensioactifs anioniques sulfates ou sulfonates,
(B) un ou plusieurs tensioactifs amphotères et
(C) une ou plusieurs silicones aminées de masse moléculaire moyenne en poids supérieure ou égale à 75000,
   le rapport en poids tensioactif anionique sulfate ou sulfonate / tensioactif amphotère / allant de 1 à 2,
   la quantité totale de tensioactifs représentant de 4 % à 35 % en poids par rapport au poids total de la composition finale.

L'invention a également pour objet l'utilisation en cosmétique des compositions ci-dessus pour le nettoyage des fibres kératiniques telles que les cheveux.

Un autre objet de l'invention concerne donc l'utilisation, en pré ou de préférence en post traitement d'une coloration d'oxydation ou d'une coloration directe des fibres kératiniques humaines et plus particulièrement des cheveux, d'une composition détergente telle que définie ci-dessus pour protéger la couleur desdites fibres kératiniques teintes artificiellement vis-à-vis du lavage.

L'invention a également pour objet un procédé pour protéger la couleur vis-à-vis du lavage des fibres kératiniques teintes artificiellement, caractérisé par le fait qu'il consiste à appliquer sur lesdites fibres au moins une composition détergente telle que définie ci-dessus.

L'invention a également pour objet un procédé pour protéger la couleur des fibres kératiniques teintes artificiellement vis-à-vis du lavage, caractérisé par le fait qu'il consiste à appliquer sur lesdites fibres, avant, pendant ou de préférence après teinture, au moins une composition détergente telle que définie ci-dessus.

On entend par « fibres kératiniques humaines » les cheveux, les poils notamment de barbe ou moustache, les cils, les sourcils et de préférence les cheveux.

On entend par « fibres kératiniques teintes artificiellement » des fibres kératiniques teintes par un procédé de coloration directe ou par un procédé de coloration d'oxydation.

On entend par « lavage », une ou plusieurs applications sur les fibres kératiniques d'une composition aqueuse rincée, le plus souvent détergente telle qu'un shampooing. Cette expression inclut également les baignades en particulier en mer ou en piscine.

Les différents objets de l'invention vont maintenant être détaillés. L'ensemble des significations et définitions des composés utilisés dans la présente invention données ci-dessous sont valables pour l'ensemble des objets de l'invention.

### (A) Tensioactifs sulfate(s) ou sulfonate(s)

Selon l'invention, les tensioactifs anioniques sulfate(s) ou sulfonate(s) sont des tensioactifs anioniques comportant au moins une fonction sulfate (-OSO₃H ou -OSO₃⁻) et/ou au moins une fonction sulfonate (-SO₃H ou (-SO₃⁻).

Les tensioactifs anioniques sulfates ou sulfonates utilisables, seuls ou mélanges, dans le cadre de la présente invention, sont les sels (en particulier sels alcalins, notamment de sodium, sels d'ammonium, sels d'amines, sels d'aminoalcools ou sels de magnésium) des alkylsulfates, alkylamidosulfates, alkyléthersulfates, alkylamidoéthersulfates, alkylaryléthersulfates, des alkyléthersulfosuccinates, des acyl iséthionates, des méthyl acyl taurates; le radical alkyle ou acyle de tous ces différents composés comportant de préférence de 8 à 24 atomes de carbone, et le radical aryle désignant de préférence un groupement phényle ou benzyle.

Le nombre moyen de groupements oxyde d'éthylène ou oxyde de propylène peut aller notamment de 2 à 50 et plus particulièrement de 2 à 10.

Parmi ces tensioactifs anioniques, on préfère utiliser les sels d'alkyléthersulfates en C₈-C₁₄ et plus particulièrement ceux en C₁₂-C₁₄. Ces sels comprennent de préférence de 2 à 5 groupements d'oxyde d'éthylène.
On utilise de préférence un agent tensioactif anionique choisi parmi les alkyl(C₁₂-C₁₄) sulfates de sodium, de triéthanolamine, de magnésium ou d'ammonium, les alkyl (C₁₂-C₁₄)éthersulfates de sodium, d'ammonium ou de magnésium oxyéthylénés à 2,2 moles d'oxyde d'éthylène, le cocoyl iséthionate de sodium, les méthyltaurates.

Les tensioactifs anioniques sulfates ou sulfonates sont généralement présents à raison de 2 % à 20 % en poids, de préférence de 4 à 15 % en poids, et plus particulièrement de 5 à 12%, mieux encore de 8 à 12% en poids par rapport au poids total de la composition.

### (B) Tensioactif(s) amphotère(s) et/ou zwittérioniques:

Les agents tensioactifs amphotères et/ou zwittérioniques peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) bétaïnes ou les alkyl (C₈-C₂₀) amidoalkyl (C₁-C₆) sulfobétaïnes.

Parmi les dérivés d'amines, on peut citer les produits décrits dans les brevets US-2 528 378 et US-2 781 354 et de structures :

R₂ -CONHCH₂CH₂ -N(R₃)(R₄)(CH₂COO-) (2)

dans laquelle : R₂ CO désigne un radical acyle en C6-C24, par exemple un radical présent dans l'huile de coprah hydrolysée, un radical octoyle, décoyle ou dodécanoyle et leurs mélanges, R₃ désigne un groupement bêta-hydroxyéthyle et R₄ un groupement carboxyméthyle ;
et

R₂-CONHCH₂CH₂-N(B)(C) (3)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂ - CHOH - SO₃H
R'₂ CO désigne un radical acyle en C6-C24, par exemple un radical présent dans l'huile de coprah hydrolysée ou l'huile de lin, un radical octoyle, décoyle ou dodécanoyle, stéaroyle ou isostéaroyle, oléoyle et leurs mélanges.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.

A titre d'exemple on peut citer le cocoamphodiacetate commercialisé sous la dénomination commerciale MIRANOL^{®} C2M concentré par la société RHODIA CHIMIE.

Selon la présente invention, on préfère plus particulièrement utiliser les agents tensio-actifs amphotères appartenant au groupe des bétaïnes tels que les alkylbétaïnes en particulier la cocobétaïne commercialisée sous la dénomination « DEHYTON AB 30 » en solution aqueuse à 30 % de MA par la société HENKEL ou les alkylamidobétaines, en particulier la cocamidopropylbétaine telles que la TEGOBETAINE^{®} F50 commercialisée par la société GOLDSCHMIDT.

Le ou les tensio-actif(s) amphotère(s) sont généralement présents dans des quantités allant de 2 à 20 % en poids, de préférence de 5 à 10 % en poids, par rapport au poids total de la composition.

Dans la composition selon l'invention le rapport en poids tensioactif anionique sulfate ou sulfonate / tensioactif amphotère va de 1 à 2. Plus particulièrement il va de 1,2 à 1,9, encore plus préférentiellement de 1,5 à 1,9.

Selon l'invention, la composition peut comprendre d'autres tensioactifs et notamment un ou plusieurs tensioactif(s) anionique(s) carboxylique(s).

Ledit(s) tensioactif(s) anionique(s) carboxylique(s) est(sont) généralement présent(s) dans des concentrations allant de 0,5 % à 10 % en poids, de préférence de 1 à 5 % en poids par rapport au poids total de la composition.

Selon l'invention, la quantité totale de tensioactifs peut représenter de 4 % à 35 % en poids, plus particulièrement de 6 à 25% en poids, de préférence de 8 % à 20 % en poids, du poids total de la composition finale.

### C- Silicone aminée

Selon l'invention, on désigne par silicone aminée toute silicone comportant au moins une amine primaire, secondaire ou tertiaire et plus particulièrement au moins une amine primaire. Les silicones aminées ne comprennent pas de groupement ammonium quaternaire.

Les silicones aminées utilisées dans la composition conforme à l'invention présentent de préférence la formule générale suivante : 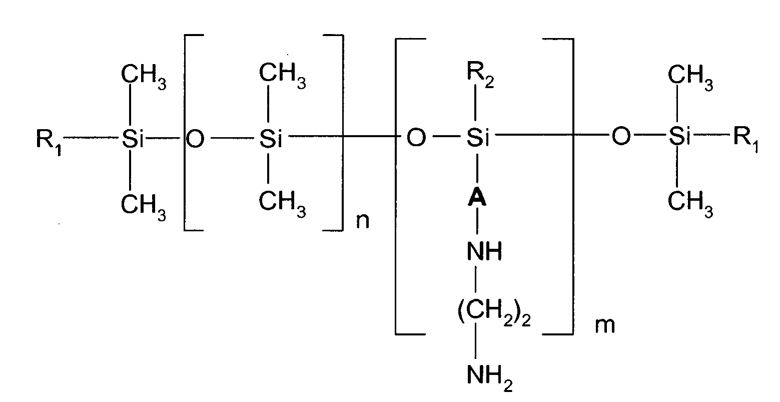 dans laquelle :
A désigne un radical alkylène linéaire ou ramifié en C₂-C₈ et de préférence en C₃.
R₁ et R₂ désignent indépendamment l'un de l'autre un radical alkyle en C₁-C₄, de préférence méthyle ou un radical alcoxy en C₁-C₄ de préférence méthoxy ou un radical hydroxy, m et n sont des nombres tels que la masse moléculaire en poids (MW) soit supérieure ou égale à 75000.
De préférence, les radicaux R1 sont identiques et désignent un radical hydroxy.

De préférence la viscosité de la silicone aminée est supérieure à 25 000 mm /s mesurée à 25°C.
Plus préférentiellement, la viscosité de la silicone aminée est comprise entre 30 000 et 200 000 mm²/s à 25°C et encore plus préférentiellement entre 50 000 et 150 000 mm²/s mesurée à 25°C et mieux encore de 70 000 à 120 000 mm²/s. Les viscosités des silicones sont par exemple mesurées selon la norme « ASTM 445 Appendice C ».
De préférence, la charge cationique de la silicone aminée est inférieure ou égale à 0,5 meq/g, allant de préférence de 0,01 à 0,1 meq/g, mieux encore de 0,03 à 0,06 meq/g.

De préférence, la silicone aminée présente une masse moléculaire moyenne en poids allant de 75000 à 1 000 000 et encore plus préférentiellement allant de 100 000 à 200 000.
Les masses moléculaires moyennes en poids de ces silicones aminées sont mesurées par Chromatographie par Perméation de Gel (GPC) à température ambiante en équivalent polystyrène. Les colonnes utilisées sont des colonnes p styragel. L'éluant est le THF, le débit est de 1 ml/mn. On injecte 200 µl d'une solution à 0,5% en poids de silicone dans le THF. La détection se fait par réfractométrie et UVmétrie.

Lorsque ces silicones aminées sont mises en oeuvre, une forme de réalisation particulièrement intéressante est leur utilisation sous forme d'émulsion huile-dans-eau.
L'émulsion huile-dans-eau peut comprendre un ou plusieurs tensioactifs. Les tensioactifs peuvent être de toute nature mais de préférence cationique et/ou non ionique.
Les particules de silicone dans l'émulsion ont un diamètre moyen en volume (D4,3) allant généralement de 10 nm à 1000 nanomètres, de préférence de 50 nm à 800 nanomètres, plus particulièrement de 100 nm à 600 nanomètres et encore plus particulièrement de 200 nm à 500 nanomètres. Ces granulométries peuvent notamment être déterminées à l'aide d'un granulomètre laser comme par exemple le Malvern Mastersizer 2000.

Une silicone aminée particulièrement préférée répondant à cette formule est par exemple la DOW CORNING 2-8299® CATIONIC EMULSION de la société DOW CORNING.

La ou les silicones aminées sont utilisées de préférence en une quantité allant de 0,01 à 20% en poids par rapport au poids total de la composition. Plus préférentiellement, cette quantité va de 0,05 à 10% en poids, encore plus particulièrement de 0,1 à 5 % en poids et mieux encore de 0,5 à 3% en poids par rapport au poids total de la composition.

Selon un mode de réalisation de l'invention, les compositions peuvent comprendre en outre un sel hydrosoluble et/ou un alcool hydrosoluble mono ou polyhydroxylé. Les sels hydrosolubles selon l'invention sont de préférence les sels de métaux mono ou divalents et d'un acide minéral ou organique.
On peut citer en particulier le chlorure de sodium, le chlorure de potassium, le chlorure de calcium, le sulfate de magnésium, le citrate de sodium, les sels de sodium de l'acide phosphorique. Le chlorure de sodium est particulièrement préféré.
Comme alcool hydrosoluble mono ou polyhydroxylé on peut citer l'éthanol, l'isopropanol, le propylèneglycol, le glycérol, l'hexylèneglycol.

Les compositions détergentes selon l'invention présentent un pH final généralement compris entre 2 et 8. De préférence, ce pH est compris entre 3 et 7,5. L'ajustement du pH à la valeur désirée peut se faire classiquement par ajout d'une base (organique ou minérale) dans la composition, par exemple de la soude, de l'ammoniaque ou une (poly)amine primaire, secondaire ou tertiaire comme la monoéthanolamine, la diéthanolamine, la triéthanolamine, l'isopropanolamine ou la propanediamine-1,3, ou encore par ajout d'un acide minéral ou organique, de préférence l'acide citrique ou l'acide chlorhydrique.

Le milieu aqueux cosmétiquement acceptable peut être constitué uniquement par de l'eau ou par un mélange d'eau et d'un solvant cosmétiquement acceptable tel que les alcools mono ou polyhydroxylés mentionnés ci-dessus ou les éthers de ces alcools, les esters, les cétones.

La composition selon l'invention comprend de préférence au moins 30% en poids d'eau et plus particulièrement de 50 à 90% en poids et encore préférentiellement de 70 à 85% en poids par rapport au poids total de la composition.

La composition comprend de préférence moins de 20% en poids de phase grasse, par rapport au poids total de la composition.

La phase grasse comprend tous les corps gras insolubles dans l'eau à température ambiante de la composition tels que notamment les esters gras, les huiles végétales, minérales, synthétiques, les alcools gras, les acides gras, les amides gras, les cires, les silicones. La phase grasse représente de préférence de 0,1 à 15% en poids et plus particulièrement de 0,5 à 10% en poids par rapport au poids total de la composition et encore plus particulièrement de 0,5 à 8% en poids.

Les compositions conformes à l'invention peuvent contenir en plus de l'association définie ci-dessus des agents régulateurs de viscosité tels que des agents épaississants. On peut citer en particulier les scléroglucanes, les gommes de xanthane, les alcanolamides d'acide gras, les alcanolamides d'acide alkyl éther carboxylique éventuellement oxyéthylénés avec jusqu'à 5 moles d'oxyde d'éthylène tel que le produit commercialisé sous la dénomination "AMINOL A15" par la société CHEM Y, les acides polyacryliques réticulés et les copolymères acide acrylique / acrylates d'alkyle en C₁₀-C₃₀ réticulés. Ces agents régulateurs de viscosité sont utilisés dans les compositions selon l'invention dans des proportions pouvant aller jusqu'à 10 % en poids par rapport au poids total de la composition. On utilisera de préférence les acides polyacryliques réticulés tels que par exemple le Carbopol 980 de NOVEON

Les compositions conformes à l'invention peuvent également contenir jusqu'à 5 % d'agents nacrants ou opacifiants bien connus dans l'état de la technique tels que par exemple les alcools gras, les palmitates de sodium ou de magnésium, les stéarates et hydroxystéarates de sodium ou de magnésium, les alcool gras, les dérivés acylés à chaîne grasse tels que les distéarates d'éthylène glycol ou de polyéthylèneglycol, les éthers à chaînes grasses tels que par exemple le distéaryléther ou le 1-(hexadécyloxy)-2-octadécanol, les cyclodextrines en particulier les β- cyclodextrines.

Les compositions conformes à l'invention peuvent éventuellement contenir en outre d'autres agents ayant pour effet d'améliorer les propriétés cosmétiques de cheveux ou de la peau sans cependant altérer la stabilité des compositions. On peut citer à ce sujet les agents tensioactifs cationiques, les polymères anioniques ou non ioniques ou cationiques ou amphotères, les protéines, les hydrolysats de protéines, les céramides, les pseudocéramides, les huiles végétales, les acides gras notamment à chaînes linéaires ou ramifiées en C₁₆-C₄₀ tels que l'acide méthyl-18 eicosanoïque, les hydroxyacides, les vitamines, les provitamines telle que le panthénol, les silicones, volatiles ou non volatiles, solubles et insolubles dans le milieu différentes des silicones aminées de formules (I) ou (II), les filtres UV, les agents hydratants, les agents antipelliculaires ou antiséborrhéiques, les agents antichute des cheveux, les agents anti-radicaux libres, et leurs mélanges.

Selon un mode particulièrement préféré, les compositions selon l'invention comprennent en outre un ou plusieurs polymères cationiques non siliconés.

Les polymères cationiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A-0 337 354 et dans les demandes de brevets français FR-A-2 270 846, 2 383 660, 2 598 611, 2 470 596 et 2 519 863.

De manière encore plus générale, au sens de la présente invention, l'expression « polymère cationique » désigne tout polymère contenant des groupements cationiques et/ou des groupements ionisables en groupements cationiques.

Les polymères cationiques utilisables selon l'invention ont de préférence une densité de charge cationique supérieure ou égale à 0,01 meq./g, et plus particulièrement allant de 0,1 à 10 meq./g et en particulier de 3 à 8 meq/g.

Parmi tous les polymères cationiques susceptibles d'être utilisés dans le cadre de la présente invention, on préfère mettre en oeuvre les dérivés d'éther de cellulose quaternaires tels que les produits commercialisés sous la dénomination « JR 400 » par la société UNION CARBIDE CORPORATION, les cyclopolymères, en particulier les homopolymères de sel de diallyldiméthylammonium en particulier le MERQUAT 100 et les copolymères de sel de diallyldiméthylammonium et d'acrylamide en particulier les chlorures, commercialisés sous les dénominations « MERQUAT 550 » et « MERQUAT S » par la société MERCK, les gommes de guar modifiées par du chlorure de 2,3-époxypropyl triméthylammonium commercialisées par exemple sous la dénomination « JAGUAR C13S » par la société RHODIA CHIMIE, les homopolymères et les copolymères éventuellement réticulés de sel de (méth)acryloyloxyéthyltriméthylammonium, vendus par exemple par la société CIBA en solution à 50% dans de l'huile minérale sous les dénominations commerciales SALCARE SC92 (copolymère réticulé du chlorure de méthacryloyloxyéthyltriméthylammonium et de l'acrylamide) et SALCARE SC95 (homopolymère réticulé du chlorure de méthacryloyloxyéthyl triméthylammonium), les copolymères quaternaires de vinylpyrrolidone et de sel de vinyl imidazole tels que les produits commercialisés par BASF sous les dénominations LUVIQUAT FC 370, LUVIQUAT FC 550, LUVIQUAT FC 905 et LUVIQUAT HM-552.

Les homopolymères et les copolymères de sels (chlorure) de diallyldimétylammomnium sont particulièrement préférés.

Selon l'invention, le ou les polymères cationiques peuvent représenter de 0,005 % à 10 % en poids, de préférence de 0,01 % à 5 % en poids, et encore plus préférentiellement de 0,1 % à 3 % en poids, du poids total de la composition finale.

Les compositions selon l'invention peuvent contenir également des synergistes de mousses tels que des 1,2-alcanediols en C₁₀-C₁₈ ou des alcanolamides gras en C₁₀-C₁₈ dérivés de mono ou de diéthanolamine.

Bien entendu, l'homme du métier veillera à choisir ce ou ces éventuels composés complémentaires et/ou leurs quantités de manière telle que la stabilité des compositions et les propriétés cosmétiques attachées intrinsèquement à la composition conforme à l'invention ne soient pas, ou substantiellement pas, altérées par la ou les adjonctions envisagées.

Selon un mode particulier de l'invention, les compositions selon l'invention sont transparentes.

La transparence peut se mesurer par mesure de la transmittance à 700nm via un spectromètre (par exemple spectromètre Lambda 14 de Perkin Elmer ou UV2101PC de Shimadzu). Les compositions transparentes ont une transmittance supérieure ou égale à 94%, de préférence de 96 à 100%.

Ces compositions peuvent se présenter sous la forme de liquides plus ou moins épaissis, de crèmes ou de gels et elles conviennent principalement au lavage, au soin des fibres kératiniques en particulier des cheveux.

L'invention a également pour objet un procédé de lavage et de conditionnement des fibres kératiniques telles que notamment les cheveux consistant à appliquer sur lesdites matières mouillées une quantité efficace d'une composition telle que définie ci-dessus, puis à effectuer un rinçage à l'eau après un éventuel temps de pause.

Les compositions selon l'invention sont utilisées de préférence comme shampooings pour le lavage et le conditionnement des cheveux et ils sont appliqués dans ce cas-là sur les cheveux humides dans des quantités efficaces pour les laver, et la mousse générée par massage ou friction avec les mains est ensuite éliminée après un éventuel temps de pause, par rinçage avec de l'eau, l'opération pouvant être répétée une ou plusieurs fois.

Des exemples concrets, mais nullement limitatifs, illustrant l'invention vont maintenant être donnés.

### EXEMPLE 1

On a réalisé la composition de shampooing conforme à l'invention

| | 1 |
|---|---|
| Lauryléthersulfate de sodium (C12/C14 à 70/30) à 2,2 moles d'oxyde d'éthylène en solution aqueuse à 70% de MA | 11,55 gMA |
| Cocoamidopropylbétaïne à 38% de MA (TEGOBETAINE F 50 de GOLDSCHMIDT) | 5,5 gMA |
| N-cocoyl amidoéthyl, N-éthoxycarboxyméthyl glycinate de sodium en solution aqueuse à 31,5% de matière active (MIRANOL C2M CONC de RHODIA CHIMIE) | 0,95 gMA |
| Silicone aminée en émulsion aqueuse à 57,5% MA (DC2-8299 de DOW CORNING) | 1,55 g MA |
| Copolymère de chlorure de poly-diméthyl diallyl ammonium et d'acrylamide dans l'eau à 8 % de matière active (MERQUAT 550 de NALCO) | 0,24 gMA |
| Acide polyacrylique réticulé (CARBOPOL 980 de NOVEON) | 0,2 g |
| Distéarate d'éthylèneglycol | 2, 5 g |
| Acide salicylique | 0,2 g |
| - Parfum, conservateur | qs |
| - Agent de pH qs pH | qs pH5,3 |
| - Eau déminéralisée qsp | 100 g |

Les cheveux traités avec ces compositions se démêlent facilement et sont légers et lisses de la racine à la pointe.
Les cheveux teints artificiellement traités avec cette composition présentent une meilleure ténacité de la couleur aux shampooings répétés.

### EXEMPLE 2

On a réalisé les compositions de shampooing suivantes :

| | 2 (invention) | A |
|---|---|---|
| Lauryléthersulfate de sodium (C12/C14 à 70/30) à 2,2 moles d'oxyde d'éthylène en solution aqueuse à 70% de MA | 11,55 gMA | 7 gMA |
| Cocobétaïne à 30% MA (DEHYTON AB 30 de COGNIS) | 6,45 gMA | 2,5 gMA |
| Silicone aminée en émulsion aqueuse à 57,5% MA (DC2-8299 de DOW CORNING) | 1,8 g MA | 1,8 g MA |
| Hydroxyéthylcellulose quaternisée par du 2,3-époxypropyl triméthylammonium (UCARE POLYMER JR 400 de AMERCHOL) | 0,4 gMA | 0,4 gMA |
| Distéarate d'éthylèneglycol | 1,5 g | 1,5 g |
| Polymère acrylique en émulsion (AQUA SF1 de NOVEON) | 0,8 gMA | 0,8 gMA |
| - Conservateur | qs | qs |
| - Acide citrique ou NaOH qs pH | 5,5 | 5,5 |
| - Eau déminéralisée qsp | 100 g | 100 g |
| | | |
| Rapport en poids tensioactif anionique sulfate ou sulfonate/ tensioactif amphotère | 1,79 | 2,8 |

Après 8 shampooings (0,4 g de shampooing par g de cheveux), on a obtenu sur des cheveux naturels 90% blancs permanentés teints par une coloration Majirouge 6.66 de l'OREAL (4g de crème et 6 g d'oxydant 20 volumes) un DE (écart de couleur avant et après traitement par les 8 shampooings) de 11,95 contre un DE de 14,05 obtenu pour les cheveux traités avec le shampooing A.
Plus le DE est petit, plus la protection est efficace.
Les cheveux teints artificiellement traités avec la composition 2 ont présenté une meilleure ténacité de la couleur aux shampooings répétés que ceux traités avec la composition A.

## Revendications

1. Composition cosmétique détergente, **caractérisée par le fait qu'**elle comprend, dans un milieu aqueux cosmétiquement acceptable,
(A) un ou plusieurs tensioactifs anioniques sulfate ou sulfonate,
(B) un ou plusieurs tensioactifs amphotères et
(C) une ou plusieurs silicones aminées de masse moléculaire moyenne en poids Mw allant de 100 000 à 200 000, ne comprenant pas de groupement ammonium quaternaire, de formule (I) suivante : 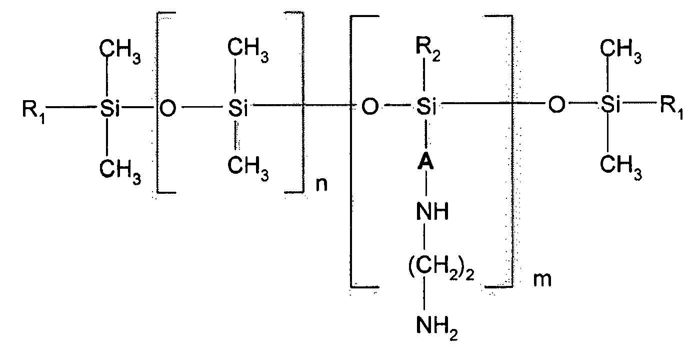 dans laquelle :
A désigne un radical alkylène linéaire ou ramifié en C₂-C₈,
m et n sont des nombres tels que la masse moléculaire en poids (Mw) va de 100 000 à 200 000, et
R₁, R₂ désignent indépendamment l'un de l'autre un radical alkyle en C₁-C₄ ou un radical alcoxy en C₁-C₄ ou un radical hydroxy;
le rapport en poids tensioactif anionique sulfate ou sulfonate/ tensioactif amphotère allant de 1 à 2, et
la quantité totale de tensioactifs représentant de 4 % à 35 % en poids par rapport au poids total de la composition finale.

2. Composition selon la revendication 1, **caractérisée en ce que** le(s) tensioactif(s) anionique(s) sulfate ou sulfonate est (sont) présent(s) dans des concentrations allant de 2 à 20 % en poids, de préférence de 4 à 15 % en poids, par rapport au poids total de la composition.

3. Composition selon l'une quelconque des revendications 1 à 2, **caractérisée en ce que** le(s) tensioactif(s) amphotère(s) est(sont) présent(s) dans des concentrations allant de 2 à 20 % en poids, de préférence de 5 à 10 % en poids, par rapport au poids total de la composition.

4. Composition selon l'une quelconque des revendications 1 à 3, **caractérisée par le fait que** le rapport en poids tensioactif anionique sulfate ou sulfonate /tensioactif amphotère va de 1,2 à 1,9, plus particulièrement de 1,5 à 1,9.

5. Composition selon l'une quelconque des revendications précédentes, **caractérisée par le fait que** A désigne un radical alkylène linéaire ou ramifié en C₃.

6. Composition selon l'une des revendications précédentes, **caractérisée en ce que** R₁ est un radical hydroxy.

7. Composition selon l'une quelconque des revendications précédentes, **caractérisée par le fait que** la viscosité de ladite silicone aminée est supérieure à 25 000 mm²/s à 25°C, de préférence allant de 30 000 à 200 000 mm²/s à 25°C et plus préférentiellement de 50 000 à 150 000 mm²/s à 25°C.

8. Composition selon l'une quelconque des revendications précédentes **caractérisée en ce que** la charge cationique de la silicone aminée est inférieure ou égale à 0,1 meq/g.

9. Composition selon l'une quelconque des revendications précédentes, **caractérisée par le fait que** lesdites silicones aminées sont présentes dans des concentrations allant de 0,01 à 20 %, et de préférence de 0,05 à 10 % en poids par rapport au poids total de la composition et encore plus particulièrement de 0,1 à 5% en poids.

10. Composition selon l'une quelconque des revendications précédente, **caractérisée en ce qu'**elle comprend en outre un ou plusieurs tensioactif(s) anionique(s) carboxylique(s).

11. Composition selon l'une quelconque des revendications précédentes, **caractérisée par le fait que** la composition comprend en outre un ou plusieurs polymères cationiques non siliconés.

12. Composition selon la revendication 11, **caractérisée par** le fait le polymère cationique est choisi parmi les homopolymères de sel de diallyldiméthylammonium.

13. Composition selon l'une quelconque des revendications 11 à 12, **caractérisée en ce que** ledit polymère cationique représente de 0,005 % à 10 % en poids, de préférence de 0,01 % à 5 % en poids, et encore plus préférentiellement de 0,1 % à 3 % en poids, du poids total de la composition finale.

14. Procédé pour protéger la couleur vis-à-vis du lavage des fibres kératiniques teintes artificiellement notamment des fibres kératiniques humaines et plus particulièrement des cheveux, **caractérisé par le fait qu'**il consiste à appliquer sur lesdites fibres, avant ou après teinture desdites fibres, au moins une composition telle définie à l'une des revendications 1 à 13.

## Patentansprüche

1. Kosmetische Reinigungszusammensetzung, **dadurch gekennzeichnet, dass** sie in einem kosmetisch unbedenklichen wässrigen Medium:
(A) ein oder mehrere anionische Sulfat- oder Sulfonat-Tenside,
(B) ein oder mehrere amphotere Tenside und
(C) ein oder mehrere Aminosilikone mit einem gewichtsmittleren Molekulargewicht Mw im Bereich von 100.000 bis 200.000, die keine quaternäre Ammoniumgruppe enthalten, der folgenden Formel (I) : 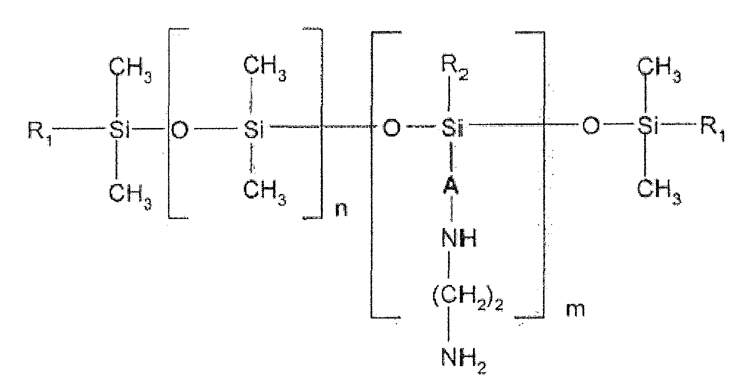 worin:
A für einen linearen oder verzweigten C₂-C₈-Alkylenrest steht,
m und n solche Zahlen sind, dass das gewichtsmittlere Molekulargewicht (Mw) 100.000 bis 200.000 beträgt, und
R₁ und R₂ unabhängig voneinander für einen C₁-C₄-Alkylrest oder einen C₁-C₄-Alkoxyrest oder einen Hydroxyrest stehen;
wobei das Gewichtsverhältnis von anionischem Sulfat- oder Sulfonat-Tensid zu amphoterem Tensid im Bereich von 1 bis 2 liegt, und
wobei die Gesamtmenge an Tensid 4 bis 35 Gew.-%, bezogen auf das Gesamtgewicht der fertigen Zusammensetzung, ausmacht, umfasst.

2. Zusammensetzung nach Anspruch 1, **dadurch gekennzeichnet, dass** das anionische Sulfat- oder Sulfonat-Tensid bzw. die anionischen Sulfat- oder Sulfonat-Tenside in Konzentrationen im Bereich von 2 bis 20 Gew.-%, vorzugsweise 4 bis 15 Gew.-%, bezogen auf das Gesamtgewicht der Zusammensetzung, vorliegt bzw. vorliegen.

3. Zusammensetzung nach einem der Ansprüche 1 bis 2, **dadurch gekennzeichnet, dass** das amphotere Tensid bzw. die amphoteren Tenside in Konzentrationen im Bereich von 2 bis 20 Gew.-%, vorzugsweise 5 bis 10 Gew.-%, bezogen auf das Gesamtgewicht der Zusammensetzung, vorliegt bzw. vorliegen.

4. Zusammensetzung nach einem der Ansprüche 1 bis 3, **dadurch gekennzeichnet, dass** das Gewichtsverhältnis von anionischem Sulfat- oder Sulfonat-Tensid zu amphoterem Tensid im Bereich von 1,2 bis 1,9 und spezieller 1,5 bis 1,9 liegt.

5. Zusammensetzung nach einem der vorhergehenden Ansprüche, **dadurch gekennzeichnet, dass** A für einen linearen oder verzweigten C₃-Alkylenrest steht.

6. Zusammensetzung nach einem der vorhergehenden Ansprüche, **dadurch gekennzeichnet, dass** R₁ ein Hydroxyrest ist.

7. Zusammensetzung nach einem der vorhergehenden Ansprüche, **dadurch gekennzeichnet, dass** die Viskosität des Aminosilikons größer als 25.000 mm²/s bei 25°C ist, vorzugsweise im Bereich von 30.000 bis 200.000 mm²/s bei 25°C liegt und weiter bevorzugt 50.000 bis 150.000 mm²/s bei 25°C beträgt.

8. Zusammensetzung nach einem der vorhergehenden Ansprüche, **dadurch gekennzeichnet, dass** die kationische Ladung des Aminosilikons kleiner gleich 0,1 meq/g ist.

9. Zusammensetzung nach einem der vorhergehenden Ansprüche, **dadurch gekennzeichnet, dass** die Aminosilikone in Konzentrationen im Bereich von 0,01 bis 20 Gew.-% und vorzugsweise 0,05 bis 10 Gew.-% und noch spezieller 0,1 bis 5 Gew.-%, bezogen auf das Gesamtgewicht der Zusammensetzung, vorliegen.

10. Zusammensetzung nach einem der vorhergehenden Ansprüche, **dadurch gekennzeichnet, dass** sie außerdem ein oder mehrere carboxylgruppenhaltige anionische Tenside umfasst.

11. Zusammensetzung nach einem der vorhergehenden Ansprüche, **dadurch gekennzeichnet, dass** die Zusammensetzung außerdem ein oder mehrere kationische Nichtsilikonpolymere umfasst.

12. Zusammensetzung nach Anspruch 11, **dadurch gekennzeichnet, dass** das kationische Polymer aus Diallyldimethylammoniumsalz-Homopolymeren ausgewählt ist.

13. Zusammensetzung nach einem der Ansprüche 11 bis 12, **dadurch gekennzeichnet, dass** das kationische Polymer 0,005 bis 10 Gew.-%, vorzugsweise 0,01 bis 5 Gew.-% und noch weiter bevorzugt 0,1 bis 3 Gew.-% des Gesamtgewichts der fertigen Zusammensetzung ausmacht.

14. Verfahren zum Schützen der Farbe künstlich gefärbter Keratinfasern, insbesondere menschlicher Keratinfasern und spezieller der Haare, gegenüber Waschen, **dadurch gekennzeichnet, dass** es daraus besteht, dass man auf die Fasern vor oder nach dem Färben der Fasern mindestens eine Zusammensetzung gemäß einem der Ansprüche 1 bis 13 aufbringt.

## Claims

1. Detergent cosmetic composition, **characterized in that** it comprises, in a cosmetically acceptable aqueous medium:
(A) one or more sulfate or sulfonate anionic surfactants,
(B) one or more amphoteric surfactants and
(C) one or more amino silicones with a weight-average molecular mass Mw ranging from 100 000 to 200 000, not comprising any quaternary ammonium groups, of formula (I) below: 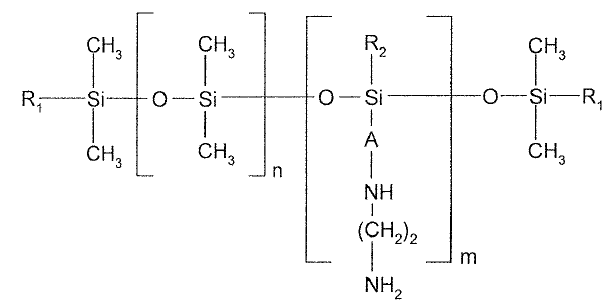 in which:
A denotes a linear or branched C₂-C₈ alkylene radical,
m and n are numbers such that the weight-average molecular mass Mw ranges from 100 000 to 200 000, and
R₁ and R₂ denote, independently of each other, a C₁-C₉ alkyl radical or a C₁-C₄ alkoxy radical or a hydroxyl radical;
the sulfate or sulfonate anionic surfactant/amphoteric surfactant weight ratio ranging from 1 to 2, and
the total amount of surfactants representing from 4% to 35% by weight relative to the total weight of the final composition.

2. Composition according to Claim 1, **characterized in that** the sulfate or sulfonate anionic surfactant(s) is (are) present in concentrations ranging from 2% to 20% by weight and preferably from 4% to 15% by weight relative to the total weight of the composition.

3. Composition according to either of Claims 1 and 2, **characterized in that** the amphoteric surfactant(s) is (are) present in concentrations ranging from 2% to 20% by weight and preferably from 5% to 10% by weight relative to the total weight of the composition.

4. Composition according to any one of Claims 1 to 3, **characterized in that** the sulfate or sulfonate anionic surfactant/amphoteric surfactant weight ratio ranges from 1.2 to 1.9 and more particularly from 1.5 to 1.9.

5. Composition according to any one of the preceding claims, **characterized in that** A denotes a linear or branched C₃ alkylene radical.

6. Composition according to one of the preceding claims, **characterized in that** R₁ is a hydroxyl radical.

7. Composition according to any one of the preceding claims, **characterized in that** the viscosity of the said amino silicone is greater than 25 000 mm²/s, preferably ranging from 30 000 to 200 000 mm²/s at 25°C and more preferentially from 50 000 to 150 000 mm²/s at 25°C.

8. Composition according to any one of the preceding claims, **characterized in that** the cationic charge of the amino silicone is less than or equal to 0.1 meq./g.

9. Composition according to any one of the preceding claims, **characterized in that** the said amino silicones are present in concentrations ranging from 0.01% to 20%, preferably from 0.05% to 10% by weight and even more particularly from 0.1% to 5% by weight relative to the total weight of the composition.

10. Composition according to any one of the preceding claims, **characterized in that** it also comprises one or more carboxylic anionic surfactant(s).

11. Composition according to any one of the preceding claims, **characterized in that** the composition also comprises one or more non-silicone cationic polymers.

12. Composition according to Claim 11, **characterized in that** the cationic polymer is chosen from diallyldimethylammonium salt homopolymers.

13. Composition according to either of Claims 11 and 12, **characterized in that** the said cationic polymer represents from 0.005% to 10% by weight, preferably from 0.01% to 5% by weight and even more preferentially from 0.1% to 3% by weight relative to the total weight of the final composition.

14. Process for protecting the colour with respect to washing of artificially dyed keratin fibres, especially human keratin fibres and more particularly the hair, **characterized in that** it consists in applying to the said fibres, before or after dyeing the said fibres, at least one composition as defined in one of Claims 1 to 13.
